# EUROPEAN PATENT APPLICATION

(11) **EP 3 848 346 A1**
(43) Date of publication of application: **14.07.2021**
(21) Application number: 19858584.6
(22) Date of filing: 03.09.2019
(51) Int. Cl.: C07C 29/76, C07C 29/86, C07C 31/20

(54) **METHOD FOR REFINING NON-PETROLEUM-BASED ETHYLENE GLYCOL**

(30) Priority: 05.09.2018 CN 201811032489
(71) Applicant: Changchun Meihe Science and Technology Development Co., Ltd., Changchun, Jilin 130102 (CN)
(72) Inventor: YUAN, Yi, Changchun, Jilin 130102 (CN)
(74) Representative: Abthorpe, Mark
(86) International application number: PCT/CN2019/104167
(87) International publication number: WO 2020/048444

(57) **Abstract**

The invention provides a process for refining non-petroleum based ethylene glycol, wherein impurities having a boiling point close to that of ethylene glycol are separated. In the process, C₅-C₂₀ oleophilic alcohol compounds, C₅-C₂₀ alkanes and/or C₄-C₂₀ oleophilic ketone compounds are subjected to azeotropism as an azeotropic agent together with the non-petroleum based ethylene glycol to obtain an azeotrope containing ethylene glycol. Then the azeotropic agent in the azeotrope is separated and removed to obtain an ethylene glycol crude product which is further purified to obtain ethylene glycol.

## Description

### Technical Field

The invention relates to a process for refining ethylene glycol, in particular relates to a process for refining non-petroleum based ethylene glycol comprising impurities including butanediol, pentanediol, hexanediol, and optional 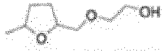 etc. which have a boiling point close to that of ethylene glycol, and impurities including a trace of acids, ethers, aldehydes, ketones and/or alcohols etc. which affect the ultraviolet transmittance of ethylene glycol.

### Background Art

In recent years, technologies of non-petroleum routes, such as coal-to-ethylene glycol and a production of ethylene glycol from raw materials of biomass, have developed rapidly because of the uncertainty of oil prices and people's attention to sustainable development. However, by-products different from those in the petroleum routes to produce ethylene glycol, such as alcohol impurities including butanediol, pentanediol, hexanediol, 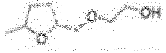 and the like, and impurities including a trace or even an amount of below the detection limit of gas chromatography of acids, ethers, aldehydes, ketones and/or alcohols etc. which affect the ultraviolet transmittance of ethylene glycol, are produced during the production of ethylene glycol in non-petroleum routes due to differences in synthetic routes. A traditional method for purification of liquid-phase compounds is a rectification process for separation by using different boiling points of substances. However, the boiling points of these impurities are close to that of ethylene glycol. For example, alcohol impurities such as butanediol, hexanediol, pentanediol 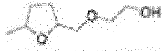 and the like, and impurities including a trace or even an amount of below the detection limit of gas chromatography of acids, ethers, aldehydes, ketones and/or alcohols etc. which affect the ultraviolet transmittance of ethylene glycol have similar physical properties to ethylene glycol and the boiling points are very close to that of ethylene glycol. Therefore, a separation of ethylene glycol from the alcohol impurities by a direct rectification method would lead to a low distillation yield of ethylene glycol and a high energy consumption. Moreover, the ultraviolet transmittance of ethylene glycol obtained by rectification cannot directly satisfy the requirements of fiber grade and bottle grade polyesters as it still contains some trace impurities.

US4935102, US4966658, US5423955 and US8906205 all describe technologies of separating ethylene glycol from butanediol by using different azeotropic agents. An azeotropic agent has an azeotropic point with ethylene glycol. Generally, the temperature of an azeotropic point is apparently lower than the boiling point of ethylene glycol. Thus, a distinct temperature difference is produced between the boiling point of an azeotrope of ethylene glycol and an azeotropic agent and that of impurities such as butanediol. The separation of ethylene glycol and butanediol can be achieved economically by means of rectification.

The process of producing ethylene glycol in non-petroleum routes will produce alcohol impurities besides ethylene glycol, such as pentanediol, hexanediol, 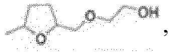 which have a boiling point very close to that of ethylene glycol, and impurities including a trace or even an amount of below the detection limit of gas chromatography of acids, ethers, aldehydes, ketones and/or alcohols which affect the ultraviolet transmittance of ethylene glycol. However, the above-mentioned several literatures only describe the effects of separation of ethylene glycol from butanediol by using an azeotropic agent without mentioning the effects of separation of ethylene glycol from pentanediol, hexanediol, 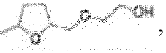 etc. after using an azeotropic agent. Nor do they mention the effects of separation of ethylene glycol from a trace or even an amount of below the detection limit of gas chromatography of acids, ethers, aldehydes, ketones and/or alcohols impurities which affect the ultraviolet transmittance of ethylene glycol. Therefore, these patents do not mention that the ultraviolet transmittance of ethylene glycol can be improved.

CN106946654A describes an adsorption bed with porous carbon adsorbents for adsorbing impurities in biomass-derived ethylene glycol to achieve the effects of refining ethylene glycol. This technique only describes the improvement of the ultraviolet transmittance of ethylene glycol but fails to describe that it can separate butanediol, a compound having the following molecular formula 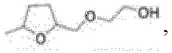 pentanediol, hexanediol and other alcohol impurities.

### Contents of the invention

The invention provides a process for refining non-petroleum based ethylene glycol, in which impurities having a boiling point close to that of ethylene glycol are separated. The process can increase the purity of said ethylene glycol to 99.90% or more, preferably 99.95% or more under the conditions of a high recovery rate of ethylene glycol of 95% or more, preferably 97% or more and particularly preferably 98% or more. Moreover, the ultraviolet transmittances of the obtained ethylene glycol at a wavelength of 220nm, 275nm and 350 nm are improved to 75% or more, 92% or more and 99% or more respectively.

Said non-petroleum based ethylene glycol refers to the ethylene glycol produced in non-petroleum routes, especially ethylene glycol produced from coal or biomass. It comprises, but not limited to, ethylene glycol, butanediol, pentanediol and hexanediol. Preferably, the non-petroleum based ethylene glycol further comprises a compound having the following molecular formula: 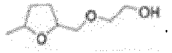 Said butanediol is preferably 1,2-butanediol, said pentanediol is preferably 1,2-pentanediol, and said hexanediol is preferably 1,2-hexanediol.

In the process of the invention, one, two or more of C₅-C₂₀ oleophilic alcohol compounds, C₅-C₂₀ alkanes and C₄-C₂₀ oleophilic ketone compounds are subjected to azeotropism as an azeotropic agent together with the non-petroleum based ethylene glycol to obtain an azeotrope containing ethylene glycol, then water is added to dissolve the ethylene glycol in the azeotrope, the water-insoluble azeotropic agent is separated from the ethylene glycol aqueous solution, and ethylene glycol is obtained from dehydration and refining of the resulting ethylene glycol aqueous solution.

In one embodiment of the invention, the C₅-C₂₀ oleophilic alcohol compounds are preferably C₆-C₁₅ oleophilic alcohol compounds, more preferably C₇-C₁₂ oleophilic alcohol compounds and particularly preferably C₇-C₁₀ oleophilic alcohol compounds. The oleophilic alcohol compounds may be aliphatic alcohols and alcohols containing heterocycles. For example, examples of the oleophilic alcohol compounds are pentanol and its isomers, hexanol and its isomers, heptanol and its isomers, octanol and its isomers, nonanol and its isomers, decanol and its isomers, undecanol and its isomers, lauryl alcohol and its isomers, and benzyl alcohol. Especially preferably, said oleophilic alcohol compounds are heptanol, isoheptanol, octanol, isooctanol, nonanol, isononanol, decanol and isodecanol.

In another embodiment of the invention, the C₅-C₂₀ alkanes are preferably C₅-C₁₅ alkanes, preferably C₅-C₁₂ alkanes and particularly preferably C₅-C₁₀ alkanes. The alkanes may be straight-chain alkanes, branched alkanes, cycloalkanes or alkanes containing a benzene ring. For example, examples of the alkanes are pentane and its isomers, hexane and its isomers, heptane and its isomers, octane and its isomers, nonane and its isomers, decane and its isomers, undecane and its isomers, dodecane and its isomers, cyclopentane and cyclohexane, ethylbenzene and its isomers. Especially preferably, the alkanes are hexane, heptane, octane, nonane, decane, undecane, dodecane, cyclopentane, cyclohexane and ethylbenzene.

In another embodiment of the invention, the said C₄-C₂₀ oleophilic ketone compounds are preferably C₅-C₁₅ oleophilic ketone compounds, more preferably C₆-C₁₂ oleophilic ketone compounds, particularly preferably C₆-C₁₀ oleophilic ketone compounds. The ketones may be aliphatic ketones or alicyclic ketones. Especially preferably, the ketones are heptanone, diisobutyl ketone, cyclohexanone and 2-nonanone.

The biomass according to the invention preferably refers to edible first generation biomass including corn, sugarcane, etc., and non-food second generation biomass of agricultural and forestry wastes including straw, timber, bagasse, etc.. Preferably, the non-petroleum based ethylene glycol of the invention comprises, but not limited to, ethylene glycol, butanediol (preferably 1,2-butanediol), pentanediol (preferably 1,2-pentanediol), hexanediol (preferably 1,2-hexanediol) and 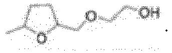 The non-petroleum based ethylene glycol of the invention optionally comprises propylene glycol, glycerol and/or sorbitol. More preferably, said non-petroleum based ethylene glycol comprises but not limited to:
1-100 wt.% of ethylene glycol (excluding end point of 100 wt.% ), preferably 1-99 wt.% of ethylene glycol, more preferably 5-99 wt.% of ethylene glycol and particularly preferably 10-95 wt.% of ethylene glycol;
0-95 wt.%, preferably 0-50 wt.%, more preferably 0-30 wt.%, particularly preferably 0-10 wt.% of butanediol (preferably 1,2-butanediol, excluding end point of 0);
0-95 wt.%, preferably 0-50 wt.%, more preferably 0-10 wt.%, particularly preferably 0-1 wt.% of pentanediol (preferably 1,2-pentanediol, excluding end point of 0);
0-95 wt.%, preferably 0-50 wt.%, more preferably 0-10 wt.%, particularly preferably 0-1 wt.% of hexanediol (preferably 1,2-hexanediol, excluding end point of 0), and
optionally 0-95 wt.%, preferably 0-50 wt.%, more preferably 0-10 wt.%, particularly preferably 0-1 wt.% of

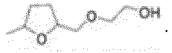

Said non-petroleum based ethylene glycol further optionally comprises:
0-95 wt.%, preferably 0.1-50 wt.% of 1,2-propanediol,
0-50 wt.%, preferably 0.01-10 of wt.% 2,3-butanediol,
0-20 wt.%, preferably 0.01-10 wt.% of glycerol, and/or
0-20 wt.%, preferably 0.01-10 wt.% of sorbitol.

In the process of the invention, the azeotropic agent forms an azeotrope by azeotropism with ethylene glycol. There is a distinct temperature difference between the boiling point of the azeotrope and that of impurities such as butanediol, pentanediol, hexanediol, 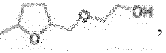 and a trace of other acids, ethers, aldehydes, ketones and/or alcohols etc. that affect the ultraviolet transmittance. Therefore, ethylene glycol can be economically purified, for example, by a rectification process.

The azeotropic agent can be separated from an aqueous solution containing ethylene glycol by an extraction process after mixing the azeotrope with water. Said aqueous solution containing ethylene glycol is refined after dehydration to obtain ethylene glycol.

### Description of Figures

Fig. 1 is a flowchart of azeotropically refining process of the non-petroleum based ethylene glycol of the invention.
Fig. 2 is a flowchart of traditional rectification process of non-petroleum based ethylene glycol.

### Mode of carrying out the invention

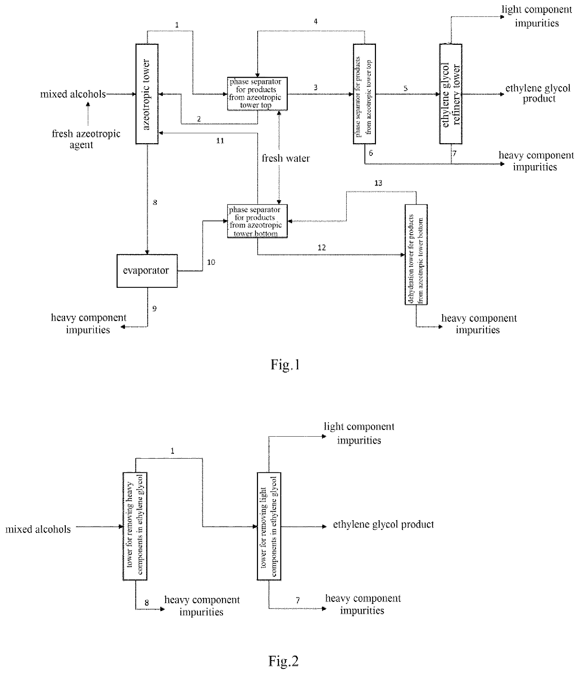

In combination with Fig. 1, the refining process of the invention is described as follows:
A mixed alcohol feed and an azeotropic agent feed are mixed before entering the azeotropic tower, where the azeotropic tower is a rectification tower. The weight ratio of the azeotropic agent feed to ethylene glycol of the mixed alcohol feed is 0.1:1-20:1, preferably 0.2:1-10:1 and more preferably 0.5:1-10:1. The operating pressure of the azeotropic tower is 1 kPa (absolute) - 101 kPa (absolute), and the weight ratio of the reflux material to the extracted material in the azeotropic tower (i.e., reflux ratio) is 0.1:1-15:1. Therein, most of the ethylene glycol and a small amount of other impurities in the mixed alcohol feed are extracted from the top of the azeotropic tower together with the azeotropic agent (i.e., stream 1) and enter a phase separator for products from azeotropic tower top. The heavy components impurities including, but not limited to, butanediol, pentanediol, hexanediol and optional 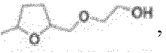 and a small amount of azeotropic agent are extracted from the azeotropic tower bottom (i.e., stream 8) and enter the evaporator.

Steam 1 and fresh water and optional recycled water (i.e., stream 4) are mixed and stratified in the phase separator for products from azeotropic tower top. An azeotropic agent layer (i.e., stream 2) is recycled to the azeotropic tower, while water layer (i.e., stream 3) enters a dehydration tower for products from azeotropic tower top.

In the dehydration tower for products from azeotropic tower top, the water in stream 3 is extracted from the top of the tower (i.e., stream 4) and recycled to the phase separator for products from azeotropic tower top. Ethylene glycol containing light component impurities (i.e., stream 5) is extracted from the side line and enters the ethylene glycol refinery tower. The heavy component impurities (i.e., stream 6) in the tower bottom are discharged from the system.

Stream 5 is refined for purification of ethylene glycol in the ethylene glycol refinery tower, and the ethylene glycol is extracted from the side line of the refinery tower. Both the purity and ultraviolet transmittance of the obtained ethylene glycol product satisfy the requirements of fiber-grade and bottle-grade polyesters. The other light component impurities are extracted from the top of the ethylene glycol refinery tower. The heavy component impurities are extracted from the bottom of the ethylene glycol refinery tower.

The materials in the bottom of the azeotropic tower (i.e., stream 8) enter the evaporator, wherein the heavy component impurities having an extremely high boiling point, such as glycerol and sorbitol, are separated from the bottom of the evaporator and discharged from the system (i.e., stream 9).

Stream 10 comprising, but not limited to, an azeotropic agent, butanediol, pentanediol, hexanediol and optional 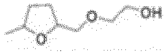 enters the phase separator for products from the azeotropic tower bottom, and then is mixed with fresh water and optional recycled water (i.e., stream 13) and then stratified. Therein, the azeotropic agent layer (i.e., stream 11) is recycled to the azeotropic tower while the water layer (i.e., stream 12) comprising, but not limited to, water, butanediol, pentanediol and hexanediol enters the dehydration tower for products from azeotropic tower bottom for dehydration.

The water in the water layer (i.e., stream 12) of the phase separator for products from azeotropic tower bottom is separated in the dehydration tower for products from azeotropic tower bottom, extracted from the top of the tower (i.e., stream 13) and then recycled to the phase separator for products from azeotropic tower bottom. Impurities comprising, but not limited to, butanediol, pentanediol and hexanediol are extracted from the bottom of the dehydration tower for products from azeotropic tower bottom and discharged from the system.

The technology of the invention can separate the ethylene glycol in the non-petroleum based ethylene glycol from the impurities comprising, but not limited to, butanediol, pentylene glycol, hexanediol and optional 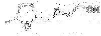 under the condition of a high recovery rate of ethylene glycol of 95% or more, preferably 97% or more, and particularly preferably 98% or more. In the meanwhile, the purity of ethylene glycol is improved to 99.90% or more, preferably 99.95% or more, and the ultraviolet transmittances of the obtained ethylene glycol are improved to 75% or more, 92% or more and 99% or more at a wavelength of 220 nm, 275 nm and 350 nm respectively. Hence, the problem that the separation of impurities such as butanediol, pentanediol, hexanediol and optional 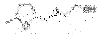 cannot be simultaneously achieved together with the improvement of ultraviolet transmittance in the prior art technology of purification of non-petroleum based ethylene glycol is solved.

### Examples

The present invention is further described by the following examples. However, the present invention is not limited thereto.

### Example 1

According to the flowchart illustrated in Fig.1, the mixed alcohol feed was the material obtained from the dehydration and the removal of the light components of the mixed product produced from the raw material of biomass. The material was composed of, in percentage by weight, 85.1% of ethylene glycol, 6.6% of 1,2-propanediol, 2.2% of 1,2-butanediol, 0.4% of 2,3-butanediol, 0.7% of 1,4-butanediol, 0.2% of 1,2-pentanediol, 0.2% of 1,2-hexanediol, 0.1% of 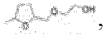 0.5% of glycerol, 0.5% of sorbitol, and 3.5% of other light and heavy components.

The mixed alcohol feed and the fresh azeotropic agent isooctanol were mixed and entered the 45^{th} theoretical plate of the azeotropic tower. The weight ratio of the azeotropic agent (including fresh azeotropic agent and recycled azeotropic agent stream 2 and stream 11) to ethylene glycol in the mixed alcohol feed was 3.39:1. There were altogether 90 theoretical plates in the azeotropic tower. The recycled azeotropic agent stream 2 from the tower top and the recycled azeotropic agent stream 11 from the tower bottom entered the azeotropic tower from the 40^{th} theoretical plate of the azeotropic tower respectively. The operating pressure of the azeotropic tower was 50 kPa (absolute), and the reflux ratio was 0.5:1. Stream 1 from the top tower separated by the azeotropic tower was composed of an azeotropic agent, ethylene glycol, 1,2-propanediol, 1,2-butanediol, 2,3-butanediol, 1,4-butanediol, 1,2-pentanediol, 1,2-hexanediol, 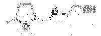 and other light components, respectively in percentage by weight of 74.97%, 22.18%, 2.54%, 0.11%, 0.08%, 0%, 0%, 0%, 0% and 0.12%.

Stream 9 of heavy components having a high boiling point was separated from stream 8 by an evaporator.

Stream 10 and stream 13 from the top of the dehydration tower for products from azeotropic tower bottom entered the phase separator for products from azeotropic tower bottom. The stratified azeotropic agent layer (i.e., stream 11) which was a recycled azeotropic agent was recycled to the azeotropic tower; the water layer (i.e., stream 12) which was a mixture of alcohol and water entered the dehydration tower for products from azeotropic tower bottom for dehydration and the water (i.e., stream 13) was recycled to the phase separator for products from azeotropic tower bottom.

Stream 1 from the top of the azeotropic tower together with stream 4 from the top of the dehydration tower for products from azeotropic tower top entered the phase separator for products from azeotropic tower top. After separation by the phase separator, the water layer stream (i.e., stream 3) entered the dehydration tower for products from azeotropic tower top for dehydration. After dehydration, the side-line stream 5 entered the 60^{th} theoretical plate of the ethylene glycol refinery tower. The ethylene glycol refinery tower had a total of 90 theoretical plates with a reflux ratio of 20:1 and an operating pressure of 10 kPa (absolute). The ethylene glycol product was extracted from the 80^{th} theoretical plate of the ethylene glycol refinery tower. By respectively analyzing via the method of the national standard GB/T4649-2008 and ASTM E2409 and ASTM E2139 of the USA, the purity of the refined ethylene glycol in percentage by weight was 99.96%, and the ultraviolet transmittances were 83.2% at a wavelength of 220 nm, 96.0% at a wavelength of 275 nm and 99.0% at a wavelength of 350 nm respectively. The total rectification yield of ethylene glycol was 98.2%.

### Example 2

According to the flowchart illustrated in Fig.1, the mixed alcohol feed was the material obtained from the dehydration and the removal of the light components of the mixed product produced from the raw material of biomass. The material was composed of, in percentage by weight, 23.2% of ethylene glycol, 55.09% of 1,2-propanediol, 4.60% of 1,2-butanediol, 1.40% of 2,3-butanediol, 0.60% of 1,4-butanediol, 0.31% of 1,2-pentanediol, 0.49% of 1,2-hexanediol, 0.15% of 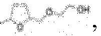 2.10% of glycerol, 1.90% of sorbitol, and 10.16% of other light and heavy components.

The mixed alcohol feed and the fresh azeotropic agent 2-nonanone were mixed and entered the 30^{th} theoretical plate of the azeotropic tower. The weight ratio of the azeotropic agent (including fresh azeotropic agent and recycled azeotropic agent stream 2 and stream 11) to ethylene glycol in the mixed alcohol feed was 7.04:1. There were altogether 90 theoretical plates in the azeotropic tower. The recycled azeotropic agent stream 2 from the tower top and the recycled azeotropic agent stream 11 from the tower bottom entered the azeotropic tower from the 25^{th} theoretical plate of the azeotropic tower respectively. The operating pressure of the azeotropic tower was 30 kPa (absolute), and the reflux ratio was 2.5:1. Stream 1 from the top tower separated by the azeotropic tower was composed of an azeotropic agent, ethylene glycol, 1,2-propanediol, 1,2-butanediol, 2,3-butanediol, 1,4-butanediol, 1,2-pentanediol, 1,2-hexanediol, 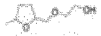 and other light components, respectively in percentage by weight of 64.96%, 9.23%, 24.98%, 0.20%, 0.32%, 0%, 0%, 0%, 0% and 0.31%.

Stream 9 of heavy components having a high boiling point was separated from stream 8 by an evaporator.

Stream 10 and stream 13 from the top of the dehydration tower for products from azeotropic tower bottom entered the phase separator for products from azeotropic tower bottom. The stratified azeotropic agent layer (i.e., stream 11) which was a recycled azeotropic agent was recycled to the azeotropic tower; the water layer (i.e., stream 12) which was a mixture of alcohol and water entered the dehydration tower for products from azeotropic tower bottom for dehydration and the water (i.e., stream 13) was recycled to the phase separator for products from azeotropic tower bottom.

Stream 1 from the top of the azeotropic tower together with stream 4 from the top of the dehydration tower for products from azeotropic tower top entered the phase separator for products from azeotropic tower top. After separation by the phase separator, the water layer stream (i.e., stream 3) entered the dehydration tower for products from azeotropic tower top for dehydration. After dehydration, the side-line stream 5 entered the 60^{th} theoretical plate of the ethylene glycol refinery tower. The ethylene glycol refinery tower had a total of 90 theoretical plates with a reflux ratio of 20:1 and an operating pressure of 10 kPa (absolute). The ethylene glycol product was extracted from the 80^{th} theoretical plate of the ethylene glycol refinery tower. By respectively analyzing via the method of the national standard GB/T4649-2008 and ASTM E2409 and ASTM E2139 of the USA, the purity of the refined ethylene glycol in percentage by weight was 99.95%, and the ultraviolet transmittances were 76.1% at a wavelength of 220 nm, 95.5% at a wavelength of 275 nm and 99.0% at a wavelength of 350 nm respectively. The total rectification yield of ethylene glycol was 98.8%.

### Example 3

According to the flowchart illustrated in Fig.1, the mixed alcohol feed was the material obtained from the dehydration and the removal of the light components of the mixed product produced from the raw material of biomass. The material was composed of, in percentage by weight, 92.50% of ethylene glycol, 4.89% of 1,2-propanediol, 1.42% of 1,2-butanediol, 0.17% of 2,3-butanediol, 0.12% of 1,4-butanediol, 0.06% of 1,2-pentanediol, 0.24% of 1,2-hexanediol, 0.07% of 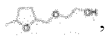 and 0.53% of other light and heavy components.

The mixed alcohol feed and the fresh azeotropic agent n-decanol were mixed and entered the 30^{th} theoretical plate of the azeotropic tower. The weight ratio of the azeotropic agent (including fresh azeotropic agent and recycled azeotropic agent stream 2 and stream 11) to ethylene glycol in the mixed alcohol feed was 0.60:1. There were altogether 90 theoretical plates in the azeotropic tower. The recycled azeotropic agent stream 2 from the tower top and the recycled azeotropic agent stream 11 from the tower bottom entered the azeotropic tower from the 25^{th} theoretical plate of the azeotropic tower respectively. The operating pressure of the azeotropic tower was 20 kPa (absolute), and the reflux ratio was 3:1. Stream 1 from the top tower separated by the azeotropic tower was composed of an azeotropic agent, ethylene glycol, 1,2-propanediol, 1,2-butanediol, 2,3-butanediol, 1,4-butanediol, 1,2-pentanediol, 1,2-hexanediol, 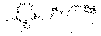 and other light components, respectively in percentage by weight of 35.81%, 60.45%, 3.15%, 0.44%, 0.02%, 0%, 0%, 0%, 0%, 0.13%.

Stream 9 of heavy components having a high boiling point was separated from stream 8 by an evaporator.

Stream 10 and stream 13 from the top of the dehydration tower for products from azeotropic tower bottom entered the phase separator for products from azeotropic tower bottom. The stratified azeotropic agent layer (i.e., stream 11) which was a recycled azeotropic agent was recycled to the azeotropic tower; the water layer (i.e., stream 12) which was a mixture of alcohol and water entered the dehydration tower for products from azeotropic tower bottom for dehydration and the water (i.e., stream 13) was recycled to the phase separator for products from azeotropic tower bottom.

Stream 1 from the top of the azeotropic tower together with stream 4 from the top of the dehydration tower for products from azeotropic tower top entered the phase separator for products from azeotropic tower top. After separation by the phase separator, the water layer stream (i.e., stream 3) entered the dehydration tower for products from azeotropic tower top for dehydration. After dehydration, the side-line stream 5 entered the 60^{th} theoretical plate of the ethylene glycol refinery tower. The ethylene glycol refinery tower had a total of 90 theoretical plates with a reflux ratio of 40:1 and an operating pressure of 20 kPa (absolute). The ethylene glycol product was extracted from the 80^{th} theoretical plate of the ethylene glycol refinery tower. By respectively analyzing via the method of the national standard GB/T4649-2008 and ASTM E2409 and ASTM E2139 of the USA, the purity of the refined ethylene glycol in percentage by weight was 99.96%, and the ultraviolet transmittances were 76.0% at a wavelength of 220 nm, 95.4% at a wavelength of 275 nm and 99.0% at a wavelength of 350 nm respectively. The total rectification yield of ethylene glycol was 96.5%.

### Example 4

According to the process illustrated in Fig. 1, the mixed alcohol feed was the same as the mixed alcohol feed in Example 3.

The mixed alcohol feed and the fresh azeotropic agent 2-heptanol were mixed and entered the 30^{th} theoretical plate of the azeotropic tower. The weight ratio of the azeotropic agent (including fresh azeotropic agent and recycled azeotropic agent stream 2 and stream 11) to ethylene glycol in the mixed alcohol feed was 8.35:1. There were altogether 90 theoretical plates in the azeotropic tower. The recycled azeotropic agent stream 2 from the tower top and the recycled azeotropic agent stream 11 from the tower bottom entered the azeotropic tower from the 25^{th} theoretical plate of the azeotropic tower respectively. The operating pressure of the azeotropic tower was 50 kPa (absolute), and the reflux ratio was 3:1. Stream 1 from the top tower separated by the azeotropic tower was composed of an azeotropic agent, ethylene glycol, 1,2-propanediol, 1,2-butanediol, 2,3-butanediol, 1,4-butanediol, 1,2-pentanediol, 1,2-hexanediol, 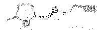 and other light components, respectively in percentage by weight of 88.15%, 11.21%, 0.55%, 0%, 0%, 0%, 0%, 0%, 0%, 0.09%.

Stream 9 of heavy components having a high boiling point was separated from stream 8 by an evaporator.

Stream 10 and stream 13 from the top of the dehydration tower for products from azeotropic tower bottom entered the phase separator for products from azeotropic tower bottom. The stratified azeotropic agent layer (i.e., stream 11) which was a recycled azeotropic agent was recycled to the azeotropic tower; the water layer (i.e., stream 12) which was a mixture of alcohol and water entered the dehydration tower for products from azeotropic tower bottom for dehydration and the water (i.e., stream 13) was recycled to the phase separator for products from azeotropic tower bottom.

Stream 1 from the top of the azeotropic tower together with stream 4 from the top of the dehydration tower for products from azeotropic tower top entered the phase separator for products from azeotropic tower top. After separation by the phase separator, the water layer stream (i.e., stream 3) entered the dehydration tower for products from azeotropic tower top for dehydration. After dehydration, the side-line stream 5 entered the 60^{th} theoretical plate of the ethylene glycol refinery tower. The ethylene glycol refinery tower had a total of 90 theoretical plates with a reflux ratio of 20:1 and an operating pressure of 20 kPa (absolute). The ethylene glycol product was extracted from the 80^{th} theoretical plate of the ethylene glycol refinery tower. By respectively analyzing via the method of the national standard GB/T4649-2008 and ASTM E2409 and ASTM E2139 of the USA, the purity of the refined ethylene glycol in percentage by weight was 99.96%, and the ultraviolet transmittances were 76.6% at a wavelength of 220 nm, 92.1% at a wavelength of 275 nm and 99.5% at a wavelength of 350 nm respectively. The total rectification yield of ethylene glycol was 97.0%.

### Example 5

According to the process illustrated in Fig. 1, the mixed alcohol feed was the same as the mixed alcohol feed in Example 3.

The mixed alcohol feed and the fresh azeotropic agent n-octane were mixed and entered the 30^{th} theoretical plate of the azeotropic tower. The weight ratio of the azeotropic agent (including fresh azeotropic agent and recycled azeotropic agent stream 2 and stream 11) to ethylene glycol in the mixed alcohol feed was 9.1:1. There were altogether 63 theoretical plates in the azeotropic tower. The recycled azeotropic agent stream 2 from the tower top and the recycled azeotropic agent stream 11 from the tower bottom entered the azeotropic tower from the 25^{th} theoretical plate of the azeotropic tower respectively. The operating pressure of the azeotropic tower was 101 kPa (absolute), and the reflux ratio was 5:1. Stream 1 from the top tower separated by the azeotropic tower was composed of an azeotropic agent, ethylene glycol, 1,2-propanediol, 1,2-butanediol, 2,3-butanediol, 1,4-butanediol, 1,2-pentanediol, 1,2-hexanediol, 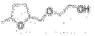 and other light components, respectively in percentage by weight of 89.55%, 9.86%, 0.51%, 0.01%, 0.01%, 0%, 0%, 0%, 0%, 0.06%.

Stream 9 of heavy components having a high boiling point was separated from stream 8 by an evaporator.

Stream 10 and stream 13 from the top of the dehydration tower for products from azeotropic tower bottom entered the phase separator for products from azeotropic tower bottom. The stratified azeotropic agent layer (i.e., stream 11) which was a recycled azeotropic agent was recycled to the azeotropic tower; the water layer (i.e., stream 12) which was a mixture of alcohol and water entered the dehydration tower for products from azeotropic tower bottom for dehydration and the water (i.e., stream 13) was recycled to the phase separator for products from azeotropic tower bottom.

Stream 1 from the top of the azeotropic tower together with stream 4 from the top of the dehydration tower for products from azeotropic tower top entered the phase separator for products from azeotropic tower top. After separation by the phase separator, the water layer stream (i.e., stream 3) entered the dehydration tower for products from azeotropic tower top for dehydration. After dehydration, the side-line stream 5 entered the 60^{th} theoretical plate of the ethylene glycol refinery tower. The ethylene glycol refinery tower had a total of 90 theoretical plates with a reflux ratio of 40:1 and an operating pressure of 20 kPa (absolute). The ethylene glycol product was extracted from the 80^{th} theoretical plate of the ethylene glycol refinery tower. By respectively analyzing via the method of the national standard GB/T4649-2008 and ASTM E2409 and ASTM E2139 of the USA, the purity of the refined ethylene glycol in percentage by weight was 99.96%, and the ultraviolet transmittances were 75.3% at a wavelength of 220 nm, 93.0% at a wavelength of 275 nm and 99.2% at a wavelength of 350 nm respectively. The total rectification yield of ethylene glycol was 97.1%.

### Example 6

According to the process illustrated in Fig. 1, the mixed alcohol feed was a mixed product produced from the raw material of coal. The material was composed of, in percentage by weight, 77.94% of ethylene glycol, 0.86% of 1,2-propanediol, 17.15% of 1,2-butanediol, 0.60% of 2,3-butanediol, 0.01% of 1,4-butanediol, 0.02% of 1,2-pentanediol, 0.01% of 1,2-hexanediol, and 3.41% of other light and heavy components.

The mixed alcohol feed and the fresh azeotropic agent isooctanol were mixed and entered the 30^{th} theoretical plate of the azeotropic tower. The weight ratio of the azeotropic agent (including fresh azeotropic agent and recycled azeotropic agent stream 2 and stream 11) to ethylene glycol in the mixed alcohol feed was 3.26:1. There were altogether 90 theoretical plates in the azeotropic tower. The recycled azeotropic agent stream 2 from the tower top and the recycled azeotropic agent stream 11 from the tower bottom entered the azeotropic tower from the 25^{th} theoretical plate of the azeotropic tower respectively. The operating pressure of the azeotropic tower was 77 kPa (absolute), and the reflux ratio was 2:1. Stream 1 from the top tower separated by the azeotropic tower was composed of an azeotropic agent, ethylene glycol, 1,2-propanediol, 1,2-butanediol, 2,3-butanediol, 1,4-butanediol, 1,2-pentanediol, 1,2-hexanediol and other light components, respectively in percentage by weight of 76.07%, 23.35%, 0.15%, 0.03%, 0.23%, 0%, 0%, 0%, 0.17%.

Stream 9 of heavy components having a high boiling point was separated from stream 8 by an evaporator.

Stream 10 and stream 13 from the top of the dehydration tower for products from azeotropic tower bottom entered the phase separator for products from azeotropic tower bottom. The stratified azeotropic agent layer (i.e., stream 11) which was a recycled azeotropic agent was recycled to the azeotropic tower; the water layer (i.e., stream 12) which was a mixture of alcohol and water entered the dehydration tower for products from azeotropic tower bottom for dehydration and the water (i.e., stream 13) was recycled to the phase separator for products from azeotropic tower bottom.

Stream 1 from the top of the azeotropic tower together with stream 4 from the top of the dehydration tower for products from azeotropic tower top entered the phase separator for products from azeotropic tower top. After separation by the phase separator, the water layer stream (i.e., stream 3) entered the dehydration tower for products from azeotropic tower top for dehydration. After dehydration, the side-line stream 5 entered the 60^{th} theoretical plate of the ethylene glycol refinery tower. The ethylene glycol refinery tower had a total of 90 theoretical plates with a reflux ratio of 20:1 and an operating pressure of 20 kPa (absolute). The ethylene glycol product was extracted from the 80^{th} theoretical plate of the ethylene glycol refinery tower. By respectively analyzing via the method of the national standard GB/T4649-2008 and ASTM E2409 and ASTM E2139 of the USA, the purity of the refined ethylene glycol in percentage by weight was 99.98%, and the ultraviolet transmittances were 77.1% at a wavelength of 220 nm, 95.0% at a wavelength of 275 nm and 99.2% at a wavelength of 350 nm respectively. The total rectification yield of ethylene glycol was 98.5%.

### Comparative Example 1

The material obtained from the dehydration and the removal of light-components of the mixed product produced from the raw material of biomass in Example 1 was used as the mixed alcohol raw material. Separation was carried out in the traditional rectification method as illustrated in Fig.2. Since no azeotropic agent was added in the traditional rectification process and no extraction section was also required, there was no need for a phase separator for products from the tower top, a phase separator for products from the tower bottom, a dehydration tower for products from the tower top, a dehydration tower for products from the tower bottom and an evaporator. Compared with Example 1, the total theoretical plates and the operating conditions of the tower for removing heavy components in ethylene glycol were the same as those of the azeotropic tower; the total theoretical plates and the operating conditions of the tower for removing light components in ethylene glycol in Comparative Example 1 were the same as those of the ethylene glycol refinery tower of Example 1. The ethylene glycol product was composed of ethylene glycol, 1,2-propanediol, 1,2-butanediol, 2,3-butanediol, 1,4-butanediol, 1,2-pentanediol and 1,2-hexanediol and 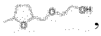 in percentage by weight of 99.45%, 0%, 0.25%, 0%, 0%, 0.02%, 0.21% and 0.07%, respectively. The ultraviolet transmittances were 56.1% at a wavelength of 220 nm, 87.2% at a wavelength of 275 nm and 96.8% at a wavelength of 350 nm. The total rectification yield of lowly pure ethylene glycol was 93.0%.

The experimental results show that the traditional rectification without an azeotropic agent cannot effectively separate impurities of 1,2-butanediol, 1,2-pentanediol, 1,2-hexanediol and optional 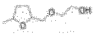 etc. in ethylene glycol. Increase of the reflux ratio and energy consumption is needed to reach the purity of 99.9%. Moreover, the ultraviolet transmittance cannot be effectively improved. The process of the invention can effectively increase the purity of said ethylene glycol to 99.90% or more under the condition of a high yield of ethylene glycol. Moreover, the ultraviolet transmittances of the obtained ethylene glycol at a wavelength of 220 nm, 275 nm and 350 nm can be increased to 75% or more, 92% or more and 99% or more respectively.

## Claims

1. A process for refining a non-petroleum based ethylene glycol, wherein one, two or more of C₅-C₂₀ oleophilic alcohol compounds, C₅-C₂₀ alkanes and C₄-C₂₀ oleophilic ketone compounds are subjected to azeotropism as an azeotropic agent together with the non-petroleum based ethylene glycol to obtain an azeotrope containing ethylene glycol, then water is added to dissolve the ethylene glycol in the azeotrope, the water-insoluble azeotropic agent is separated from the ethylene glycol aqueous solution, and ethylene glycol is obtained from dehydration and refining of the resulting ethylene glycol aqueous solution.

2. Process according to claim 1, wherein the C₅-C₂₀ oleophilic alcohol compounds are C₆-C₁₅ oleophilic alcohol compounds, preferably C₇-C₁₂ oleophilic alcohol compounds and particularly preferably C₇-C₁₀ oleophilic alcohol compounds, and the oleophilic alcohol compounds may be aliphatic alcohols and alcohols containing heterocycles, for example, pentanol and its isomers, hexanol and its isomers, heptanol and its isomers, octanol and its isomers, nonanol and its isomers, decanol and its isomers, undecanol and its isomers, lauryl alcohol and its isomers, and benzyl alcohol.

3. Process according to claim 2, wherein the C₅-C₂₀ oleophilic alcohol compounds are hexanol, isohexanol, heptanol, isoheptanol, octanol, isooctanol, nonanol, isononanol, decanol and isodecanol.

4. Process according to any of claims 1 to 3, wherein the C₅-C₂₀ alkanes are C₅-C₁₅ alkanes, preferably C₅-C₁₂ alkanes and particularly preferably C₅-C₁₀ alkanes, and the alkanes may be straight-chain alkanes, branched alkanes, cycloalkanes or alkanes containing benzene rings, for examples, pentane and its isomers, hexane and its isomers, heptane and its isomers, octane and its isomers, nonane and its isomers, decane and its isomers, undecane and its isomers, dodecane and its isomers, cyclopentane, cyclohexane, ethylbenzene and its isomers, preferably, hexane, heptane, octane, nonane, decane, undecane, dodecane, cyclopentane, cyclohexane, ethylbenzene.

5. Process according to any of claims 1 to 4, wherein the C₄-C₂₀ oleophilic ketone compounds are C₅-C₁₅ oleophilic ketone compounds, preferably C₆-C₁₂ oleophilic ketone compounds and particularly preferably C₆-C₁₀ oleophilic ketone compounds, and the ketones may be aliphatic ketones or alicyclic ketones, preferably heptanone, diisobutyl ketone, cyclohexanones, 2-nonone.

6. Process according to any of claims 1 to 5, wherein the non-petroleum based ethylene glycol is ethylene glycol produced from coal or ethylene glycol produced from biomass, wherein the biomass preferably refers to edible first generation biomass including corn, sugarcane, etc., and non-food second generation biomass of agricultural and forestry wastes including straw, timber, bagasse, etc..

7. Process according to any of claims 1 to 6, wherein the non-petroleum based ethylene comprises, but not limited to, ethylene glycol, butanediol (preferably 1,2-butanediol), pentanediol (preferably 1,2-pentanediol), hexanediol (preferably 1,2-hexanediol), and preferably further comprises

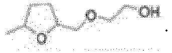

8. Process according to any of claims 1 to 7, wherein the non-petroleum based ethylene comprises propylene glycol, glycerol and/or sorbitol.

9. Process according to any of claims 1 to 8, wherein the said non-petroleum based ethylene glycol comprises,
1-100 wt.% of ethylene glycol excluding end point of 100 wt.%, preferably 1-99 wt.% of ethylene glycol, more preferably 5-99 wt.% of ethylene glycol and particularly preferably 10-95 wt.% of ethylene glycol;
0-95 wt.%, preferably 0-50 wt.%, more preferably 0-30 wt.%, particularly preferably 0-10 wt.% of butanediol, preferably 1,2-butanediol, excluding end point of 0;
0-95 wt.%, preferably 0-50 wt.%, more preferably 0-10 wt.%, particularly preferably 0-1 wt.% of pentanediol, preferably 1,2-pentanediol, excluding end point of 0;
0-95 wt.%, preferably 0-50 wt.%, more preferably 0-10 wt.%, particularly preferably 0-1 wt.% of hexanediol, preferably 1,2-hexanediol, excluding end point of 0, and;
0-95 wt.%, preferably 0-50 wt.%, more preferably 0-10 wt.%, particularly preferably 0-1 wt.% of

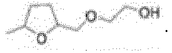

10. Process according to any of claims 1 to 9, wherein said non-petroleum based ethylene glycol comprises,
0-95 wt.%, preferably 0.1-50 wt.% of 1,2-propanediol;
0-50 wt.%, preferably 0.01-10 of wt.% 2,3-butanediol;
0-20 wt.%, preferably 0.01-10 wt.% of glycerol, and/or;
0-20 wt.%, preferably 0.01-10 wt.% of sorbitol.

11. Process according to any of claims 1 to 10, wherein said non-petroleum based ethylene glycol comprises impurities including a trace or even an amount of below the detection limit of gas chromatography of acids, ethers, aldehydes, ketones and/or alcohols etc. which affect the ultraviolet transmittance of ethylene glycol.
